(19)

Europäisches
Patentamt
European
Patent Office
Office européen
des brevets

(11) **EP 4 012 043 A1**

(12) **EUROPEAN PATENT APPLICATION**
published in accordance with Art. 153(4) EPC

(43) Date of publication:
**15.06.2022 Bulletin 2022/24**

(21) Application number: **20850436.5**

(22) Date of filing: **26.06.2020**

(51) International Patent Classification (IPC):
***C12Q 1/24*** *(2006.01)*

(52) Cooperative Patent Classification (CPC):
**C12Q 1/24**

(86) International application number:
**PCT/KR2020/008353**

(87) International publication number:
**WO 2021/025291 (11.02.2021 Gazette 2021/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.08.2019 KR 20190096059**

(71) Applicants:
• **Postech Academy-Industry Foundation
Gyeongsangbuk-do, 37673 (KR)**

• **Exosomeplus Inc.
Suwon-si, Gyeonggi-do 16229 (KR)**

(72) Inventors:
• **PARK, Jae Sung
Pohang-si, Gyeongsangbuk-do 37673 (KR)**
• **CHO, Si Woo
Suwon-si Gyeonggi-do 16494 (KR)**
• **SHIN, Hyun Woo
Busan 49395 (KR)**

(74) Representative: **Schnappauf, Georg
ZSP Patentanwälte PartG mbB
Hansastraße 32
80686 München (DE)**

(54) **METHOD FOR ISOLATING BIO NANOPARTICLES BY USING AQUEOUS TWO-PHASE SYSTEM SEPARATION COMPOSITION**

(57)     Disclosed is a method for isolating bio nanoparticles by using an aqueous two-phase system phase separation composition, the method being capable of isolating high-purity nano-sized bio nanoparticles from a biological specimen without loss thereof and damage thereto.

[FIG. 1]

EP 4 012 043 A1

**Description**

**Technical Field**

CROSS-REFERENCE TO RELATED APPLICATIONS

This application claims priority under 35 U.S.C. §119 to Korean Patent Application No. 10-2019-0096059, filed on August 7, 2019, in the Korean Intellectual Property Office, the disclosure of which is incorporated herein by reference in its entirety.

The present disclosure relates to a method for isolating bio nanoparticles by using an aqueous two-phase system phase separation composition.

**Background Art**

A biomarker is an index that can objectively measure and evaluate a normal biological process, disease progression, and a drug's responsiveness to treatment method. In the past, physiological indicators such as blood pressure, body temperature, and blood sugar levels have received an attention as biomarkers, but recently genetic materials (DNA, RNA), proteins, bacteria, viruses, etc. have received an attention as biomarkers.

Biomarkers may be classified into a variety of disease screening markers, disease markers, prognostic markers, efficacy markers, and toxicity markers according to their characteristics, and one biomarker may be used for multiple utility according to the purpose. In addition, the biomarker serves not only as a disease-related marker, but is also used in various ways in relation to new drug development, such as selecting a treatment drug, shortening the clinical trial period, and determining the therapeutic effect.

Exosomes, one of the representative biomarkers, are a kind of nanometer-sized spherical vesicles that are naturally secreted from cells in the body. It is understood that the exosomes are specifically secreted for the purpose of intercellular signal transduction, and contain functionally active substances such as proteins, lipids, and RNA contained in specific cells as they are. Accordingly, it is possible to easily diagnose diseases by isolating exosomes from blood or urine, and analyzing them to analyze characteristics related to various diseases.

Exosome isolation techniques include ultra-centrifugation isolation, size exclusion, immunoaffinity isolation, microfluidics chip and polymeric method, etc.

Among them, ultra-centrifugation isolation is the most widely used method for isolating exosomes, and is the most reliable isolation method due to its simple principle. However, this method has limitations in that the yield of recovered exosomes is low, and complicated procedures and time are required to isolate exosomes and then detect the expression levels of protein and RNA. In addition, there is a disadvantage that the device itself is expensive.

Accordingly, there is an isolation method using a size-specific exclusion with fine pores, but it raised another problem that the yield after isolation was low because the exosomes were adsorbed to the pores formed at the interface.

The immunoaffinity isolation method is a method of isolating an antibody by attaching it to the extracellular vesicle, and may be isolated with very high specificity, but it is not suitable for practical diagnosis because the antibody production process takes a long time and is expensive.

In addition, the polymeric method is a technology that makes the extracellular vesicles sink by lowering the solubility of the body fluid, and it is not suitable for diagnostic use because it requires a long incubation time and the purity of the precipitate is not good due to the sinking of the protein together.

Recently, as a method for isolating exosomes, a method for isolating nanoparticles using an aqueous two-phase system wherein a phase separation of an aqueous solution occurred, has been proposed. This method is known as aqueous biphasic systems (ABS), aqueous two-phase systems (ATPS), or aqueous two-phase extraction (ATPE) method (Non-Patent Documents 1 and 2).

Korean Patent Laid-Open Publication No. 10-2016-0116802 (Patent Document 1) and *Scientific Reports* (Non-Patent Document 3) disclose a method for separating proteins and extracellular vesicles by passing a body fluid containing proteins and extracellular vesicles (e.g., exosomes) through an aqueous two-phase system. The above method carries out a centrifugation process with 100 to 5,000 × g-force after stirring (vortexing) during the separation process to perform phase separation in a micro droplet state. This method has the advantage of being able to separate the extracellular vesicles within a short time, but because the particles may randomly pass through the interface of the aqueous solution by the external force applied during the stirring process, the filtering effect does not occur properly through the interface of the aqueous solution. There is a limit to recovering the extracellular vesicles with high purity and high yield due to the loss of such particle separation. In addition, there is a problem in that bio-particles with weak structures may be destroyed during the centrifugation process, and aggregation of bio-particles is induced.

[Related Art Document]

(Patent Document 1) Korea Patent Laid-Open Publication No. 10-2016-0116802 (10.10. 2016), Method for Separating Extracellular Vesicles using Aqueous Two-Phase System.
(Non-Patent Document 1) Hamta, Afshin et al., "Application of polyethylene glycol based aqueous two-phase systems for extraction of heavy metals". Journal of Molecular Liquids. (2017) 231: 20~24.
(Non-Patent Document 2) Juan A. Asenjo et al., Aqueous two-phase systems for protein separation: A perspective Journal of Chromatography A, Volume 1218, Issue 49, 9 December 2011, Pages 8826-8835
(Non-Patent Document 3) Hyunwoo Shin et al., High-yield isolation of extracellular vesicles using an aqueous two-phase system, Scientific Reports volume5, Article number: 13103 (2015)

**DISCLOSURE**

**Technical Problem**

In the present disclosure, an aqueous two-phase system phase separation composition is used, but particles are isolated without stirring, and phase separation is induced by density difference without an additional process such as ultracentrifugation upon phase separation, and a method for isolating nanoparticles with high purity and high efficiency without damage or loss of bio-nanoparticles from biological specimen by controlling interfacial tension of each phase wherein the phase separation occurs, was developed.

Accordingly, an object of the present disclosure is to provide a method capable of isolating bio-nanoparticles by controlling the tension at the interface where two phases are in contact in a phase separation composition comprising an aqueous two-phase system.

**Technical Solution**

In order to solve the above problem, the present disclosure provides a method of preparing a first aqueous solution and a second aqueous solution constituting an aqueous two-phase system, and separating impurities and bio-nanoparticles in a biological specimen using the composition.

According to one embodiment of the present disclosure, (a) impurities in the biological specimen are transferred to a second aqueous solution phase and bio-nanoparticles remain in the first aqueous solution phase by using an aqueous two-phase system phase separation composition to separate impurities and bio nanoparticles in the biological specimen.

According to another embodiment of the present disclosure, (b) bio-nanoparticles excluding impurities in the biological specimen are transferred to the second aqueous solution phase by using the aqueous two-phase system phase separation composition, and thus, the impurities in the biological specimen are separated from bio-nanoparticles.

In this case, processes of (a) and (b) are performed without stirring and ultracentrifugation processes. In particular, the present disclosure is characterized to design an aqueous two-phase system phase separation composition of so that the tension ($\gamma$) at the interface of the first aqueous solution phase and the second aqueous solution phase satisfies the following Equation 1 in order to separate impurities in a biological specimen from bio-nanoparticles.

$$[\text{Equation 1}]$$

$$2 \times 10^{-7} \text{ J/m2} \leq \gamma \leq 50 \times 10^{-5} \text{ J/m}^2$$

The first aqueous solution phase flows with a bulk form in the second aqueous solution phase, that is a continuous phase by gravity or buoyancy, and impurities or bio-nanoparticles in the first aqueous solution phase are transferred to the second aqueous solution phase by diffusion, and thus, impurities are separated from bio-nanoparticles.

In this case, the biological specimen may be one selected from the group consisting of cell culture fluid, blood, plasma, serum, intraperitoneal fluid, semen, amniotic fluid, breast milk, saliva, bronchoalveolar fluid, tumor effluent, tears, runny nose and urine.

In addition, bio-nanoparticles that may be separated from the biological specimen are one selected from the group consisting of extracellular vesicles including exosomes, ectosomes, viruses, proteins, LDL, HDL, external vesicle microvesicles, microparticles, apoptotic cells, membrane particles, membrane vesicles, exosome-like vesicles, and ectosome-like vesicles, etc.; biomolecules including lipid, enzyme, enzyme substrate, inhibitor, ligand, receptor, antigen, antibody, hapten, albumin, insulin, collagen, protein A, protein G, avidin, biotin, streptavidin, peptide, polypeptide, modified peptide, nucleic acid, lectin, carbohydrates, amino acids, peptide nucleic acid (PNA), locked nucleic acid (LNA), RNA, DNA, bacteria, viruses, etc.; heterogeneous biomolecules including antibody-antigen, protein A-antibody, protein G-antibody, nucleic acid-nucleic acid hybrid, aptamer-biomolecule, avidin-biotin, streptavidin-biotin, lectin-carbohydrate, lectin-glycoprotein.

**Advantageous Effects**

An aqueous two-phase system phase separation composition according to the present disclosure may recover bio-nanoparticles from a biological specimen with high purity.

In addition, since a process such as ultracentrifugation is unnecessary during the separation process, there is an advantage in that almost no damage to the bio-nanoparticles and no loss occurs. Moreover, since separation is possible in about 20 minutes, there is an advantage in that separation is possible in a short time compared to the existing ultracentrifugation process performed for 2 to 4 hours.

Conventionally, an ultracentrifugation process is performed for the separation of proteins and exosomes, but the separation is not easy because there is little difference in density between both of them. Accordingly, since the aqueous two-phase system phase separation composition of the present disclosure enables separation according to the critical particle size, and separation according to the size difference between the protein (<10 nm) and the exosome (50 nm ~ 500 nm) is possible, there is an advantage in that the protein and exosome may be separated with high purity.

Since bio-nanoparticles separated in such a high yield are recovered with high purity, they may be used as themselves without separate treatment in various bio or medical fields including diagnosis of various diseases, thereby effectively performing a role as a biomarker.

**Brief Description of Drawings**

FIG. 1 is a schematic diagram for illustrating the separation mechanism of an aqueous two-phase system phase separation composition presented in the present disclosure.

FIG. 2 is a schematic diagram showing energy barriers in a first aqueous solution phase and a second aqueous solution phase.

FIG. 3 is a simulation result showing movement according to particle size using three types of bead particles having different particle sizes.

FIG. 4 is a schematic diagram showing movement according to particle size using three types of bead particles having different particle sizes of FIG. 3.

FIG. 5 is a schematic diagram showing the energy barrier according to the interfacial tension and particle size of the first aqueous solution phase and the second aqueous solution phase.

FIG. 6 is a graph showing the change in the critical particle size according to the interfacial tension of the first aqueous solution phase and the second aqueous solution phase.

FIG. 7 is a flowchart for illustrating the isolation method of bio-nanoparticles presented in the present disclosure.

FIG. 8 is a graph showing a change in the escape velocity at the interface according to the particle size.

FIG. 9 is a graph showing a change in a critical particle size according to a change in temperature.

FIG. 10 is a photograph showing the movement of fluorescent beads after forming the first aqueous solution phase on the second aqueous solution phase in Example.

FIG.11 is an image showing the degree of movement of fluorescent beads with time, and at the bottom thereof, the interfacial tension between the first aqueous solution phase (DEX) and the second aqueous solution phase (PEG) of A, B, and C is shown.

FIG. 12 is a graph showing the separation capacity of extracellular vesicle/protein according to temperature, with (a) the recovery efficiency of the extracellular vesicle, and (b) the protein removal rate.

FIG. 13 is a graph showing the separation capacity of extracellular vesicle/protein according to time, with (a) the recovery efficiency of the extracellular vesicle, and (b) the protein removal rate.

FIG. 14a is a graph showing changes in the amount of RNA or the number of particles (NTA) related to the recovery efficiency of extracellular vesicles according to the methods of Example 1, Comparative Example 1 and Comparative Example 2, and FIG. 14b is a graph showing purity.

FIG. 15a is a graph showing the protein removal rates according to the methods of Example 1, Comparative Example 1, and Comparative Example 2, and FIG. 15b is a graph showing the results of measuring the number of extracellular vesicles per ug of protein by CD63 ELISA.

FIG. 16 is an image obtained by photographing the recovered extracellular vesicles with a transmission electron microscope, with (a) the extracellular vesicles recovered in Example 1, and (b) the extracellular vesicles recovered in Comparative Example 1.

FIG. 17 is an image obtained by photographing the recovered extracellular vesicles with a transmission electron microscope, with (a) the extracellular vesicles recovered in Example 1, and (b) the extracellular vesicles recovered in Comparative Example 3.

**Best Mode**

The present disclosure provides a method for separating impurities and bio-nanoparticles from a biological specimen containing the impurities and bio-nanoparticles.

The biological specimen referred to herein is a material derived from a living organism, and may be interpreted to include all materials defined as living organisms, including animals, plants, microorganisms, viruses, and fungi. Preferably, it refers to a specimen obtained from a biological subject, including a specimen of biological tissue or fluid origin obtained in vivo or in vitro. Such specimen may be, but are not limited to, tissues, fractions, fluids, and cells isolated from mammals, including humans. More specifically, the biological specimen is a material that may be a sample for diagnosing a disease, and may be one selected from the group consisting of cell culture solution that may be dissolved in water, blood, plasma, serum, intraperitoneal fluid, semen, amniotic fluid, breast milk, saliva, bronchoalveolar fluid, tumor effusion, tears, runny nose, and urine. Such a biological specimen includes proteins.

The size of the biological specimen is in the range of 1 to 500 nm, preferably 3 to 450 nm, more preferably 5 to 400 nm, still more preferably 5 to 350 nm, still more preferably 10 to 250 nm, and most preferably 30 to 180 nm.

The bio-nanoparticle referred to herein may be a subcellular organelle such as cells, organelles, microorganisms, endotoxins and substructures of microorganisms such as aggregates or an extracellular vesicle, which are included in the biological specimen.

Preferably, the "extracellular vesicle (EV)" refers to a nano-sized extracellular vesicle secreted from a cell and released into the extracellular space. The extracellular vesicles are divided into the inside and outside by a lipid double membrane composed of cell membrane components, and have cell membrane lipids, membrane proteins, genetic materials, and cytoplasmic components of the cell, enabling indirect understanding of the properties and conditions of cells. In addition, the extracellular vesicle acts as an extracellular messenger mediating a communication between cells by binding to other cells and tissues deliver membrane components, mRNAs, miRNAs, proteins (growth hormones, cytokines, etc.), etc. and delivering these transmitters to recipient cells.

Specifically, the extracellular vesicles are one selected from the group consisting of exosomes, ectosomes, exovesicles, microvesicles, microparticles, apoptotic bodies, membrane particles, membrane vesicles, exosome-like vesicles, and ectosome-like vesicles, and is preferably an exosome.

In addition, the bio-nanoparticle may be a biomolecule or a heterogeneous biomolecule other than the extracellular vesicles.

The biomolecules include lipid, enzyme, enzyme substrate, inhibitor, ligand, receptor, antigen, antibody, hapten, albumin, insulin, collagen, protein A, protein G, avidin, biotin, streptavidin, peptide, polypeptide, modified peptides, nucleic acids, lectins, carbohydrates, amino acids, peptide nucleic acids (PNA), locked nucleic acids (LNA), RNA, DNA, bacteria, viruses, and the like.

In addition, heterologous biomolecules include biomolecules, antibody-antigen, protein A-antibody, protein G-antibody, nucleic acid-nucleic acid hybrid, aptamer-biomolecule, avidin-biotin, streptavidin-biotin, lectins-carbohydrate, and lectin-glycoprotein.

More preferably, the bio-nanoparticles including the extracellular vesicles may have a diameter of 20 to 500 nm, specifically 25 to 400 nm, preferably 30 to 350 nm, more preferably 30 to 200 nm, still more preferably 50 to 200 m, most preferably, 50 to 150 nm.

Impurities referred to herein refer to all materials except for bio-nanoparticles in a biological specimen.

Various methods are used to perform a separation of impurities/bio nanoparticles from a biological specimen. However, damage or loss of bio-nanoparticles occurs during the separation process, and thus a new design for the separation process is required. For example, one of the bio-nanoparticles, exosomes, may be used as a biomarker. In a biological specimen such as blood, impurities such as proteins, cholesterol (LDL, HDL), including exosomes, are present together. The exosomes have a size larger than that of the impurities by several tens to hundreds of nm. Accordingly, when a filter capable of passing only a specific range of sizes is used, it is possible to effectively separate exosomes and impurities.

Accordingly, in the present disclosure, the bio-nanoparticles are separated with high purity without loss or damage by using the above-mentioned aqueous two-phase system phase separation composition.

Aqueous two-phase system phase separation composition

The aqueous two-phase system referred to herein means that aqueous solutions having different densities are phase-separated in a liquid-liquid state.

Therefore, the aqueous two-phase system phase separation composition presented in the present disclosure means that two types of aqueous solutions exist in a phase-separated form, and separation between them is possible through the escape of impurities or bio-nanoparticles at the interface (i.e., interface) of the phase-separated state.

Separation mechanism using aqueous two-phase system phase separation composition

A mechanism for separation of impurities/bio nanoparticles using an aqueous two-phase system phase separation composition is described as follows. In this case, since the impurities and bio-nanoparticles are particles having a nano-level size, they will be described below as the expression of nanoparticles.

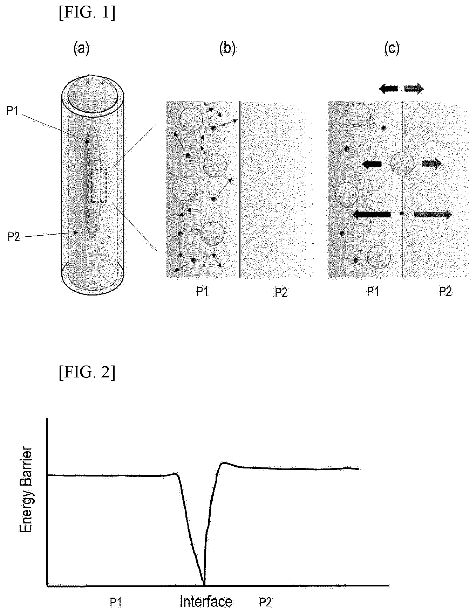

FIG. 1 is a schematic diagram for illustrating the separation mechanism of an aqueous two-phase system phase separation composition presented in the present disclosure.

First, three types of nanoparticles to be isolated are prepared. The three types of nanoparticles are for explanation, and all nanoparticles in which two or more types of nanoparticles having different particle sizes are mixed for separation may be possible.

Next, two aqueous solutions are prepared to form an aqueous two-phase system. At this time, the object that needs to be separated (mixed nanoparticles, that is, a biological specimen) is included in any one of the first aqueous solution phase P1 and the second aqueous solution phase P2. In FIG. 1, for convenience of explanation, it was mixed with a first aqueous solution phase P1.

In the formation of an aqueous two-phase system, two aqueous solutions exist, and these aqueous solutions are phase-separated into a first aqueous solution phase and a second aqueous solution phase P2 by contact as shown in FIG. 1a.

The first aqueous solution phase P1 exists in a state of flowing in the second aqueous solution phase P2 due to gravitational force or buoyancy. When the first aqueous solution phase P1 has a higher density than the second aqueous solution phase P2, it moves in the direction of gravity, but does not sink to the bottom due to buoyancy and is in a form of floating in the second aqueous solution phase P2.

In particular, the first aqueous solution phase P1 is present in the floating state of being in the form of a bulk, not in the state of being in the form of fine particles or droplets or the upper and lower layers being separated as in the upper/lower layer. In conventional particle separation using an aqueous two-phase system, a process such as vortexing is performed so that the first aqueous solution phase is split into fine particles, and nanoparticles are isolated. In comparison with this, the present disclosure performs the isolation of nanoparticles within the bulk form. In addition, in conventional aqueous two-phase system, the energy barrier of the aqueous solution interface is changed by an external force applied to the stirring (vortexing) process, and since the particles may freely pass through the interface by this external force, the separation effect due to the affinity between the particle surface and each aqueous phase is more pronounced than the effect of filtering of particles by the phase interface.

Referring to FIG. 1b, the mixed nanoparticles present in the first aqueous solution phase P1 in the bulk state actively undergo Brownian motion that moves irregularly in the aqueous phase, and come into contact with the interface between the aqueous solution phase P1 and the second aqueous solution phase P2.

In this case, the first aqueous solution phase P1 and the second aqueous solution phase P2 come into contact and are trapped at the interface, and the mixed nanoparticles move to the second aqueous solution phase P2 or the first aqueous solution phase P1 by diffusion coefficient of trapped nanoparticles or remains at the interface. As a result, as shown in FIG. 1c, some of the mixed nanoparticles of the first aqueous solution phase P1 move to the second aqueous solution phase P2 through the pores. As a result, after a certain time elapses, some of the mixed nanoparticles remain in the second aqueous solution phase P2, and the rest remain in the first aqueous solution phase P1 or the interface. Accordingly, by separating from the first aqueous solution phase P1 including the interface of the second aqueous solution phase P2, nanoparticles present in the second aqueous solution phase P2 are recovered and the nanoparticles may be separated.

In particular, in the process of moving the first aqueous solution phase P1 to the lower side by gravity after being injected into the second aqueous solution phase P2, since the first aqueous solution phase P1 continuously meets to form an interface with the new second aqueous solution phase P2, the movement of nanoparticles at the interface between two phases P1 and P2 may be accelerated while continuously occurring. In a phase-separated form into the upper/lower layer, which is one of the phase-separated forms, the continuous movement of nanoparticles may not occur because it is impossible to continuously form a new interface. In addition, in the phase-separated form of fine droplets, a new interface may be formed by stirring, etc., but the energy barrier at the interface is changed by the external force applied during the stirring process, and the particles may freely pass through the interface by this external force and thus, it is not suitable because many particles are lost through the interface during the stirring process. This method utilizes the separation effect by the affinity between the particle surface and each aqueous solution phase P1, P2 rather than the filtering effect at the interface, and separation occurs by a mechanism different from the present disclosure.

Separation of bio-nanoparticles through the aqueous two-phase system phase separation composition presented in the present disclosure is affected by the tension at the interface of the aqueous solution forming the first aqueous solution phase P1 and the second aqueous solution phase P2.

Interfacial tension refers to the tension generated at this interface when two or more different objects contact to form a layer without mixing. The first aqueous solution phase P1 and the second aqueous solution phase P2 contain different solutes and have different densities, and tension is formed at the interface due to the difference of size and physical properties of the different solutes. An energy barrier is formed on the surface that particles need to cross by this tension, and this energy barrier acts like a pore. If the magnitude of the tension is large, the energy barrier is high and thus the pores tend to be small, so that the size of the pores at the interface, that is, the pores having a threshold diameter (limitation diameter), is formed by the force. A part of the mixed nanoparticles moves to the first aqueous solution phase P1 through the pores thus formed.

The critical diameter is usually at the nm level, and the nm level may be maintained only when the interfacial tension between the first aqueous solution phase P1 and the second aqueous solution phase P2 has a certain range. That is, the small interfacial tension means that the first aqueous solution phase P1 and the second aqueous solution phase P2 may be miscible with each other, and so particle separation may not occur, and to the contrary, if the interfacial tension is large, the phase separation occurs but since the critical diameter is very small, separation of bio-nanoparticles may not be performed. The relationship between the interfacial tension and the critical diameter is a new concept that has not yet been proposed in the field of study of aqueous two-phase systems.

The numerical value of the interfacial tension set by the first aqueous solution phase P1 and the second aqueous solution phase P2 is designed by the composition of the first aqueous solution phase P1 and the second aqueous solution phase P2. The design of this composition depends on whether impurities and/or bio-nanoparticles in the biological specimen to be separated may diffuse beyond the energy barrier at the interface formed by the contact of the first aqueous solution phase P1 and the second aqueous solution phase P2.

FIG. 2 is a schematic diagram showing the energy barrier of the first aqueous solution phase P1 and the second aqueous solution phase P2.

Referring to FIG. 2, the first aqueous solution phase P1 and the second aqueous solution phase P2 are aqueous solutions containing different solutes, and energy barriers having each of different heights are present. The mixed nanoparticles present in the first aqueous solution phase P1 escape to the second aqueous solution phase P2 by crossing the energy barrier between the interface and the second aqueous solution phase P1 for particle separation. In this case, when the device of FIG. 1 is used, some nanoparticles escaping to the second aqueous solution phase P2 do not go back to the first aqueous solution phase P1. The passage of the energy barrier at the interface, that is, the escape energy in the first aqueous solution phase P1, is affected by the tension at the interface between the first aqueous solution phase P1 and the second aqueous solution phase P2.

As a surface tension of the interface increases, the height of the energy barrier that particles at the interface need to cross to escape to another phase increases, thereby reducing the critical size of particles that may escape the interface. This means that the interfacial tension of the interface may be used as a variable depending on how the phases of the first and second aqueous solutions to be used are selected.

In addition, the movement and escape of nanoparticles from the first aqueous solution phase P1 to the second aqueous solution phase P2 at the interface is explained through Fick's laws of diffusion. The fixed diffusion law is two laws representing the diffusion process in thermodynamics, and there are a first law and a second law, and in the present disclosure, it may be described as a second law (Fick's second law) related to continuous diffusion.

Particle movement was predicted using Fick's second law and the escaping rate of particles trapped at the interface. The rate at which particles trapped at the interface escape satisfies Equation 1.

$$\Gamma \equiv \frac{J}{n} = \alpha e^{-\Delta E/\kappa T} \text{----------- Equation (1)}$$

Wherein, $\Gamma$: ratio of particles escaping from the interface, J: particle flux, n: concentration of particles at the interface, $\alpha$: proportionality constant, $\Delta E$: energy difference of particles at the interface and particles escaping from the interface, $\kappa$: Boltzmann constant, T: temperature.

The energy barrier ($\Delta E$) of particles at the interface between the first aqueous solution phase P1 and the second aqueous solution phase P2 may be expressed by the following Equation 2.

$$\Delta E = \frac{\pi R^4}{\gamma_{Phase1/Phase2}}(\gamma_{Phase1/Phase2} - \gamma_{Particle/Phase2} + \gamma_{Particle/Phase1})^2 = \frac{\pi R^4}{\gamma_{Phase1/Phase2}}\left(1 - \frac{\kappa T \ln K}{4\pi R^2 \gamma_{Phase1/Phase2}}\right)^2 - \text{Equation (2)}$$

wherein, $\gamma_{Phase1/Phase2}$: tension acting at the interface of the first aqueous solution phase and the second aqueous solution phase, R: radius of the particle, $\gamma_{particle/Phase1}$: tension acting on the surface of particles in the first aqueous solution phase, $\gamma_{particle/Phase2}$: tension acting on the surface of particles in the second aqueous solution phase, $\kappa$: Boltzmann constant, T: absolute temperature, K: separation coefficient.

In the above Equation, $\Delta E$ means an energy barrier that particles at the interface need to cross to escape from the interface, and the larger the value, the lower the escape rate of the particles.

In addition, the flow of particles near the interface between the first aqueous solution phase P1 and the second aqueous solution phase P2 satisfies the following Equations.

$$J_{Phase1-interface} = k_1 C_{Phase1} - \Gamma_{Phase1} C_{interface} \quad \text{----------- Equation (3)}$$

$$J_{interface-Phase2} = \Gamma_{Phase2} C_{interface} - k_2 C_{Phase2} \quad \text{------- Equation (4)}$$

wherein, $J_{Phase1-interface}$: flow of particles between the first aqueous solution phase and the interface, $J_{Interface-Phase2}$: flow of particles between the second aqueous solution phase and the interface, $C_{Phase1}$: concentration of particles in the first aqueous solution phase near the interface, $C_{Phase2}$: concentration of particles in the second aqueous solution phase near the interface, $C_{Interface}$: concentration of particles at the interface, $\kappa1$, $\kappa2$: proportionality constant, $\Gamma_{Phase1}$: rate of particles escaping from the interface to the first aqueous solution phase, $\Gamma_{Phase2}$: ratio of particles escaping from the interface to the second aqueous solution phase.

Based on the above Equations, the flow of particles between the second aqueous solution phase P2 and the interface is the sum of flow of particles moving from the second aqueous solution phase P2 to the interface and the flow of particles escaping from the interface to the second aqueous solution phase P2. Likewise, the flow of particles between the first aqueous solution phase P1 and the interface is the sum of flow of particles moving from the first aqueous solution phase P1 to the interface and the flow of particles escaping from the interface to the first aqueous solution phase P1.

Simulation was performed by applying Equations 1, 2, 3, 4 to Fick's second law.

For the particles, three types of mixed bead particles of 10 nm, 50 nm and 100 nm were applied and the amount of bead particles escaping to the second aqueous solution phase P2 was measured to determine the governing Equation and coefficient of boundary conditions. As the first aqueous solution phase P1, an aqueous dextran solution (1% concentration) was used, and as the second aqueous solution phase P2, polyethylene glycol (3% concentration) was used. In addition, since the first aqueous solution phase P1 continuously contacts the new second aqueous solution phase P2 in the above Equation, it is assumed that the concentration of particles in the second aqueous solution phase P2 near the interface is close to zero. ($\cong Cphase1 \cong 0$). Accordingly, by measuring the amount of bead particles escaping to the second aqueous solution phase P2, the governing Equation and the coefficient of the boundary condition were determined. Based on the determined coefficients, particle separation according to particle size, filtration time, partition coefficient, temperature, and surface tension of the interface was simulated.

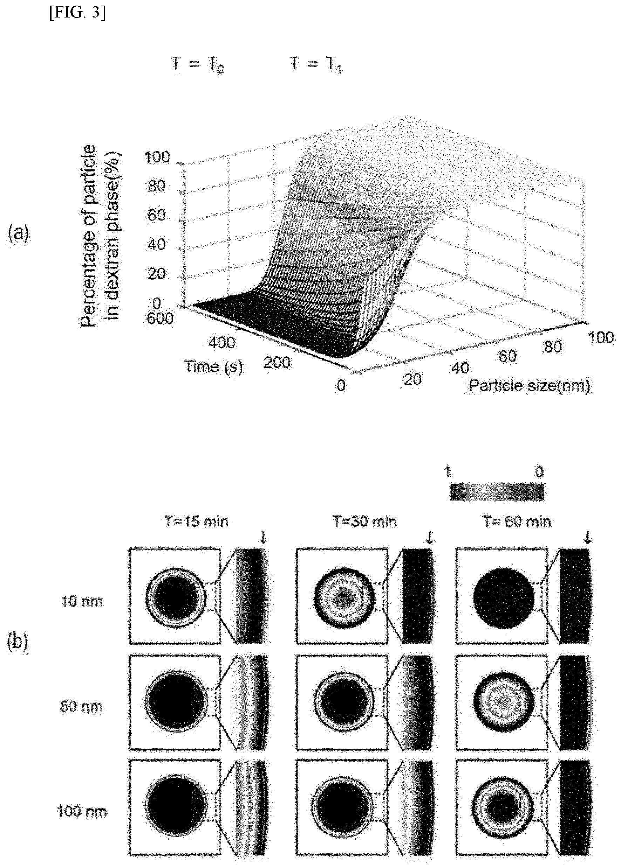

FIG. 3 is a simulation result showing movement according to particle size using three types of bead particles having different particle sizes.

Based on FIGS. 3a and 3b, the critical size of the bead particles passing through the interface gradually increased over time, and based on this, it can be seen that the small bead particles escape to the second aqueous solution phase P2.

Specifically, referring to FIG. 3a, the increase in the critical size of the bead particles that can pass through the interface over time abruptly occurs within 200 seconds, and then converges to a certain size. In addition, referring to FIG. 3b, it can be seen that in the case of nanoparticles having a size of 10 nm, all of them passed after 60 minutes, and it can be seen that 50 nm and 100 nm remain at the interface. This means that the aqueous two-phase system phase separation composition acts as a filter that only allows particles of certain size or less to pass therethrough.

In particular, in the case of 10 nm particles, it can be seen that they completely escape after 60 minutes, and it can be seen that nanoparticles may be separated with high purity, that is, a yield of 100% or close to 100%, without loss of nanoparticles from the mixed nanoparticles. From these results, when a biological specimen containing actual impurities and bio-nanoparticles is applied to the separation process, problems such as loss of bio-nanoparticles that occur in the separation process such as a conventional separation membrane are fundamentally blocked, and it can be seen that the advantage of high-purity separation of bio-nanoparticles from biological specimen may be secured by using the aqueous two-phase system phase separation composition according to the present disclosure.

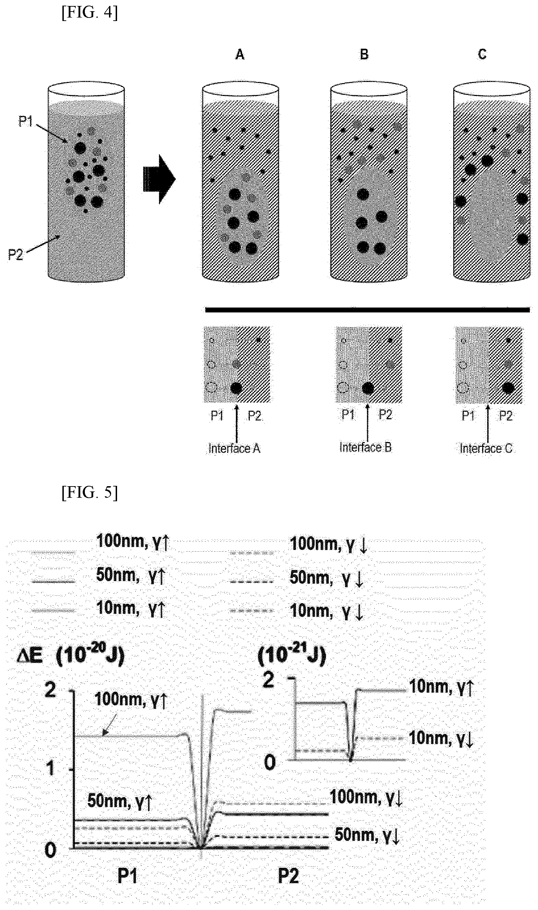

This may be seen in more detail through the schematic diagram of FIG. 4.

FIG. 4 is a schematic diagram showing movement according to particle size using three types of bead particles having different particle sizes of FIG. 3.

As shown in FIG. 4, the particles move from the first aqueous solution phase P1 to the interface formed by the contact of the second aqueous solution phase P2. Part of the particles in contact with the interface are trapped at the interface, and at this time, escape from the first aqueous solution phase P1 to the second aqueous solution phase P2 according to the size of the particles.

FIG. 4 shows three types of interfaces, in the case of Interface A, only particles of the smallest size pass through, in the case of Interface B particles up to medium size pass through, and in the case of Interface C, all of them may pass through. Through this, nanoparticles may be selectively separated from mixed nanoparticles according to particle size.

More specifically, if the smallest particle among the three particles is to be separated, it is designed like Interface A, and so the small particle may be passed through the second aqueous solution phase P2 to be recovered and separated. In addition, when the particles of the largest size are to be separated, it is designed like Interface B, and the particles of large size remaining in the first aqueous solution phase P1 may be recovered and separated. In addition, in the case of medium-sized particles, after designing as in Interface B, the second aqueous solution phase P2 is recovered, and after designing it to have Interface $\alpha$ (not shown) again, in the second aqueous solution phase P2, only medium-sized particles may be selectively recovered through a process of once again separating small-sized particles and medium-sized particles.

As mentioned above, whether the nanoparticles pass through Interface A, Interface B, and Interface C depends on the energy barrier according to each composition constituting the first aqueous solution phase P1 and the second aqueous solution phase P2, and the tension (i.e., interfacial tension) at the interface to be formed by contacting them. When the energy barrier of the first aqueous solution phase P1 and the second aqueous solution phase P2 is low, the interfacial tension formed by contacting them decreases, and as the interfacial tension decreases, the size of particles passing through the interface (i.e., critical particle size) may increase.

FIG. 5 is a schematic diagram showing the energy barrier between the first aqueous solution phase P1 and the second aqueous solution phase P2 according to interfacial tension and particle size.

In FIG. 5, the higher the interfacial tension between the first aqueous solution phase P1 and the second aqueous solution phase P2, the higher the energy barrier. In this case, there is a difference in the energy barrier that nanoparticles of each of 10 nm, 50 nm, and 100 nm needs to cross, and the energy barrier is the lowest at 10 nm. That is, the smaller the particle size, the lower the energy barrier that needs to be crossed when escaping from the interface to another phase, and if the particles exceed a specific size, the energy barrier is too high for the particles to cross.

When the first aqueous solution phase P1 and the second aqueous solution phase P2 come into contact to form an interface, escape from the nanoparticles with a size of 10 nm having a low energy barrier to the second aqueous solution phase P2 occurs first. Therefore, in order to pass the 100 nm-sized nanoparticles, the interfacial tension of the first aqueous solution phase P1 and the second aqueous solution phase P2 needs to be lowered to lower the energy barrier proportionally thereto, and thus it can be seen that escape to the second aqueous solution phase P2 may occur.

These results mean that the critical particle size through which nanoparticles may pass through the interface may be determined dominantly according to the interfacial tension. In other words, the low interfacial tension means that the size of the nanoparticles passing therethrough may be increased.

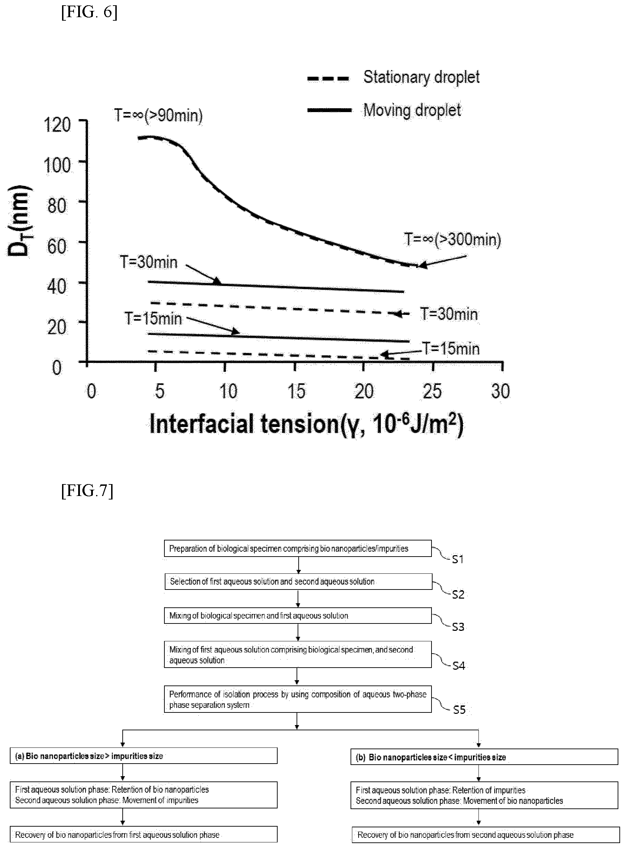

FIG. 6 is a graph showing the change in the critical particle diameter ($D_T$) according to the interfacial tension of the first aqueous solution phase P1 and the second aqueous solution phase P2.

Referring to FIG. 6, it can be seen that the critical particle size that escapes through the interface vary according to the interfacial tension.

Specifically, it can be seen that, as the interfacial tension increases, the critical particle size that may escape from the interface tends to eventually decrease. In addition, it can be seen that the critical particle size is changed according to time until reaching at a specific time, but the critical particle size finally reached is the same. In addition, it can be seen that the critical particle size increases with time, and by analyzing this, it is possible to know the time it takes to remove the desired size. For example, in the case of an aqueous two-phase system phase separation composition designed to have an interfacial tension of $5 \times 10^{-6}$ J/m$^2$, it can be seen that nanoparticles having a critical particle size of 40 nm may be separated within 30 minutes.

Through these results, in the present disclosure, in order to separate the bio-nanoparticles from a biological specimen containing impurities and bio-nanoparticles, the first aqueous solution phase P1 and the second aqueous solution phase P2 should have an interfacial tension ($\gamma$) range satisfying Equation 1 below:

[Equation 1]

$$2 \times 10^{-7} \ J/m^2 \leq \gamma \leq 50 \times 10^{-5} \ J/m^2$$

The size of pores being able to be formed by the aqueous two-phase system phase separation composition according to the present disclosure is in the range of 1 to 500 nm, preferably 3 to 450 nm, more preferably 5 to 400 nm, still more preferably 5 to 350 nm, still more preferably 10 to 250 nm, and most preferably 30 to 180 nm.

In order to have the above pores, the aqueous two-phase system phase separation composition has an interfacial tension formed at the interface in the range of $2 \times 10^{-7}$ J/m$^2$ to $50 \times 10^{-5}$ J/m$^2$, preferably $2 \times 10^{-7}$ J/m$^2$ to $40 \times 10^{-6}$ J/m$^2$, more preferably $3 \times 10^{-6}$ J/m$^2$ to $350 \times 10^{-6}$ J/m$^2$, more preferably $4 \times 10^{-6}$ J/m$^2$ to $270 \times 10^{-6}$ J/m$^2$, even more preferably from $5 \times 10^{-6}$ J/m$^2$ to $150 \times 10^{-6}$ J/m$^2$, most preferably from $10 \times 10^{-6}$ J/m$^2$ to $60 \times 10^{-6}$ J/m$^2$.

At this time, it may be seen that if the interfacial tension is not within the range shown in Equation 1, but the numerical value is decreased and out of the limit to some extent, phase separation does not occur and mixing of the first aqueous solution phase P1 and the second aqueous solution phase P2 occurs, and thus the aqueous two-phase system phase separation composition is not constituted, and to the contrary, when the interfacial tension is no less than a certain value, the escape of impurities or bio-nanoparticles does not occur. This is data that may prove that a stable aqueous two-phase system phase separation composition may be constructed only when the interfacial tension numerical range of Equation 1 presented in the present disclosure is within the range, thereby enabling separation through escape of impurities or bio-nanoparticles.

In addition, along with the separation time, it can be seen that the smaller the size of the impurities or bio-nanoparticles, the faster the rate of escaping from the interface.

According to the change of the critical particle diameter according to the interfacial tension shown in FIG. 6, it may be predicted that when the composition of the first aqueous solution phase P1, and the second aqueous solution phase P2 is designed to have a specific interfacial tension, the bio-nanoparticles are easily separated with high purity.

Preferably, the aqueous two-phase system phase separation composition according to the present disclosure may embody a filter having the range of a critical particle diameter of 1 to 500 nm, 3 to 450 nm, more preferably from 5 to 400 nm, more preferably from 5 to 350 nm, still more preferably from 10 to 250 nm, and most preferably from 30 to 180 nm, as the interfacial tension is controlled through the design of the composition, and the critical particle diameter may be controlled by adjusting the interfacial tension. In this case, the aqueous two-phase system phase separation composition may separate up to a size difference of about 10 nm between impurities and bio-nanoparticles in separation capability. In this case, the separation capability of 10 nm means that even those with a difference of 10 nm, such as 10 nm and 20 nm, in size difference between impurities and bio-nanoparticles can be separated, and separation in the case of showing a difference of 90 nm, such as 10 nm and 100 nm, may be evidently performed.

Meanwhile, FIG. 6 is a schematic diagram showing each of a case where the first aqueous solution phase P1 is a stationary phase and a flowing phase. When the time is infinite, the first aqueous solution phase P1 showed the same results in both the stationary phase and the fluidized phase, but within a predetermined time, when the first aqueous solution phase P1 has a fluidized phase, it can be seen that it is advantageous for the separation of nanoparticles. Considering that nanoparticle separation is typically performed within a predetermined time rather than an infinite time, when the first aqueous solution phase P1 has a form of a fluidized phase, it can be seen that it is preferable to apply it to the actual separation process.

Design of aqueous two-phase system phase separation composition

The tension at the interface between the first aqueous solution phase P1 and the second aqueous solution phase P2 may be achieved by designing the composition of the first aqueous solution phase and the second aqueous solution phase.

In order to have interfacial tension between the first aqueous solution phase P1 and the second aqueous solution phase P2, phase separation between them should be preceded. The phase separation may be performed with various other methods, but in the present disclosure, it may be achieved through a two-phase diagram between the first aqueous solution phase P1 and the second aqueous solution phase P2. In addition, in the separation of impurities and bio-nanoparticles, the separation rate and separation capability may be improved depending on whether these surfaces have affinity for any one of the first aqueous solution phase P1 and the second aqueous solution phase P2. In consideration of these details, a specific composition constituting the first aqueous solution phase P1 and the second aqueous solution phase P2 may be selected through a two-phase diagram to constitute an aqueous two-phase system phase separation composition.

The first aqueous solution phase P1 and the second aqueous solution phase P2 are basically aqueous solutions in which a solute is dissolved in water.

Depending on the type of solute, the first aqueous solution phase P1 and the second aqueous solution phase P2 may be an aqueous polymer solution or an aqueous salt solution in which a polymer and/or a salt are present.

The polymer as the solute may be a hydrophilic polymer.

Hydrophilic polymers that may be used may be one hydrophilic polymer selected from the group consisting of polyarginine, polylysine, polyethylene glycol, polypropylene glycol, polyethyleneimine, chitosan, protamine, polyvinyl acetate, hyaluronic acid, chondroitin sulfate, heparin, alginate, hydroxyoxypropyl methylcellulose, gelatin, starch, poly(vinyl methyl ether ether), polyvinylpyrrolidone, and combinations thereof.

In addition, the polymer as a solute may be a polymer polysaccharide. The polymer polysaccharide may be one hydrophilic polymer selected from the group consisting of cyclodextrin, glucose, dextran, mannose, sucrose, trehalose, maltose, ficoll, inositol, mannitol, sorbitol, sucrose-mannitol, glucose-mannitol, trehalose-polyethylene glycol, sucrose-polyethylene glycol, sucrose-dextran, and combinations thereof.

The salts used in the aqueous salt solution may be one hydrophilic polymer selected from the group consisting of $(NH_4)_2SO_4$, $Na_2SO_4$, $MgSO_4$, $K_2HPO_4$, $KH_2PO_4$, NaCl, KCl, NaBr, NaI, LiCl, $n$-$Bu_4NBr$, $n$-$Pr_4NBr$, $Et_4NBr$, $Mg(OH)_2$, $Ca(OH)_2$, $Na_2CO_3$, $ZnCO_3$, $Ca_3(PO_4)_2$, $ZnCh$, $(C_2H_3)_2Zn$, $ZnCO_3$, $CdCh$, $HgCh$, $CoCl_2$, $(CaNO_3)_2$, $BaCl_2$, $MgCl_2$, $PbCl_2$, $AlCl_3$, $FeCh$, $FeCl_3$, $NiCh$, AgCl, $AuCl_3$, CuCh, sodium dodecyl sulfate, sodium tetradecyl sulfate, dodecyltrimethylammonium bromide, dodecyltrmethylammonium chloride, tetradecyltrimethylammonium bromide, and combinations thereof.

In addition, a polymer salt may be used as the solute, and for example, a combination of the above-mentioned polymer and salt may be used.

The selection of the first aqueous solution phase P1 and the second aqueous solution phase P2 among the above-mentioned polymers and salts may vary depending on the characteristics (e.g., surface characteristics) of the nanoparticles to be separated, and the characteristics and concentrations that enable phase separation.

Among them, each combination that may be used as the first aqueous solution phase P1 and the second aqueous solution phase P2 may have a hydrophilicity-hydrophobic property in the case of a polymer. Since the polymer is basically dissolved in an aqueous solution, it exhibits hydrophilicity, but when the two compositions are combined, it may have relatively hydrophilicity or relatively hydrophobicity. For example, in the case of dextran and polyethylene glycol, there are characteristics that dextran has a relatively hydrophilicity and a denser molecular structure, and polyethylene glycol has relatively hydrophobicity and a less dense molecular structure. Accordingly, dextran/polyethylene glycol may be used as the first aqueous solution phase/second aqueous solution phase (P1/P2), respectively.

In addition, in the case of each combination usable as the first aqueous solution phase P1 and the second aqueous solution phase P2, molecular weight and concentration may be important selection reasons for polymers.

As the molecular weight of the polymer increases and the concentration increases, the first aqueous solution phase and the second aqueous solution phase are stably formed, and if the molecular weight of the polymer is too small, the first aqueous solution and the second aqueous solution are easily mixed.

The molecular weight of the polymer should exist in a dissolved (or swollen) state in the minimum aqueous solution, and since there is a difference in solubility in water depending on the type of polymer, it is not easy to limit the range. However, in the case of the aforementioned hydrophilic polymer, the weight average molecular weight is 200 to 2,000,000, preferably 500 to 1,000,000, more preferably 1,000 to 500,000. For example, in the case of polyethylene glycol in combination with dextran, those having a weight average molecular weight in the range of 200 to 60,000, preferably 500 to 40000 are used. In addition, the dextran has a weight average molecular weight in the range of from 15 to 1,000,000, preferably from 1,000 to 500,000.

In this case, the concentration of the polymer or the aqueous polymer salt solution may be 0.001 to 20% by weight, preferably 0.01 to 15% by weight, although there is a difference in solubility in water. If the concentration is too low, the aqueous polymer solution of the first aqueous solution phase P1 and the second aqueous solution phase P2 exhibits fluidity similar to water, and the two are miscible with each other, making it difficult to form an aqueous two-phase system. Conversely, if it is too high, it takes time to dissolve the polymer, and the tension at the interface of the aqueous two-phase system is too high, so that the critical particle diameter becomes small, making it difficult to separate nanoparticles.

When a salt is used instead of a polymer, a high concentration of salt is required to form an aqueous two-phase system. As described above, as the molecular weight of the polymer increases, the first aqueous solution phase P1 and the second aqueous solution phase P2 are stably formed, and in the case of salt, since the molecular weight is smaller than that of the polymer, an aqueous two-phase system may be formed only at high concentration. Preferably, the high concentration salt is used in an amount of 1 to 70% by weight, more preferably 5 to 50%.

When a low concentration of salt is added to a system that may already form the first aqueous solution phase and the second aqueous solution phase, the salt is dissociated into an ionic state in the aqueous solution and serves to change the movement speed of the nanoparticles. In this case, the salt preferably has an average molecular weight of 10 to 1,000 parts by weight.

Specifically, the combination of the first aqueous solution phase/the second aqueous solution phase (P1/P2) in the aqueous two-phase system phase separation composition according to the present disclosure may be used as a combination of a polymer-polymer, and a polymer-high concentration salt, as shown in Table 1 below.

[Table 1]

| | 1st aqueous phase (P1, dispersed phase) | 2nd aqueous phase (P2, continuous phase) |
|---|---|---|
| Combination of polymer-polymer | 5% dextran | 5% polyethylene glycol (MW= 100,000) |
| | 2% dextran | 5% polyvinylpyrrolidone (MW=5,000) |
| | 2% dextran | 2% polyvinyl alcohol (MW=130,000) |
| | 5% dextran | 5% ficoll (MW=400) |
| | 3% polyvinyl methyl ether (MW=5,000) | 5% polyethylene glycol (MW=35,000) |
| Combination of polymer-high concentration salt | 10% $(NH_4)_2SO_4$ | 20% polyethylene glycol (MW=35,000) |
| | 10% $Na_2SO_4$ | 20% polyethylene glycol (MW=35,000) |
| | 10% $MgSO_4$ | 20% polyethylene glycol (MW=35,000) |
| | 10% $K_2HPO_4$ | 20% polyethylene glycol (MW=35,000) |
| | 10% $KH_2PO_4$ | 20% polyethylene glycol (MW=35,000) |
| | 10% $Na_2CO_3$ | 20% polyethylene glycol (MW=35,000) |

The combination shown in Table 1 is an example, and in addition, any combination may be used as long as the various combinations using the composition as described above satisfy the interfacial tension presented in Equation 1.

**Method for isolating bio nanoparticles by using aqueous two-phase system phase separation composition**

In the present disclosure, by using the aqueous two-phase system phase separation composition as described above, impurities and bio-nanoparticles are separated from a biological specimen including impurities and bio-nanoparticles.

A first aqueous solution containing a biological specimen and a second aqueous solution are mixed to prepare the aqueous two-phase system phase separation composition where the first aqueous solution is phase-separated in the second aqueous solution, and bio-nanoparticles are separated from the biological specimen containing the impurities.

FIG. 7 is a flowchart for illustrating the isolation method of bio-nanoparticles presented in the present disclosure.

Referring to FIG. 7, first, a biological specimen including bio-nanoparticles is prepared (S1).

Next, the first aqueous solution and the second aqueous solution for designing the aqueous two-phase system phase separation composition are selected (S2).

The first aqueous solution forms a first aqueous solution phase, and the second aqueous solution forms a second aqueous solution phase. Each of these compositions is selected from the above-mentioned compositions, and the tension ($\gamma$) at the interface at which the first aqueous solution and the second aqueous solution are phase-separated is designed to satisfy Equation 1 below.

[Equation 1]

$$2 \text{ X } 10^{-7} \text{ J/m}^2 \leq \gamma \leq 500 \text{ X } 10^{-5} \text{ J/m}^2$$

Next, the first aqueous solution and the biological specimen are mixed (S3).

The biological specimen may be selected from the above. In this case, 0.0001 to 0.1 g of the biological specimen is used for 1 ml of the first aqueous solution. However, this content may vary depending on the composition of the biological specimen, and may be appropriately selected by those skilled in the art.

The biological specimen may be used, if necessary, by a known pretreatment as prior to mixing. In the example of the present disclosure, no special pretreatment was performed.

Next, the first aqueous solution containing a biological specimen and the second aqueous solution are mixed to form an aqueous two-phase system phase separation composition that is phase-separated into a first aqueous solution phase/second aqueous solution phase (S4).

The respective contents of the first aqueous solution and the second aqueous solution for constituting the aqueous two-phase system phase separation composition are mixed in a range of 0.5 to 2 ml of the second aqueous solution with respect to 1 ml of the first aqueous solution. The first aqueous solution and the second aqueous solution is appropriately mixed within the above range so that phase separation may occur stably.

Next, using the aqueous two-phase system phase separation composition prepared through (S4), a separation process is performed (S5).

In the aqueous two-phase system phase separation composition, nano-level pores through which particles can pass are formed at the interface of the first aqueous solution phase and the second aqueous solution phase, and bio-nanoparticles or impurities in the biological specimen move through these pores.

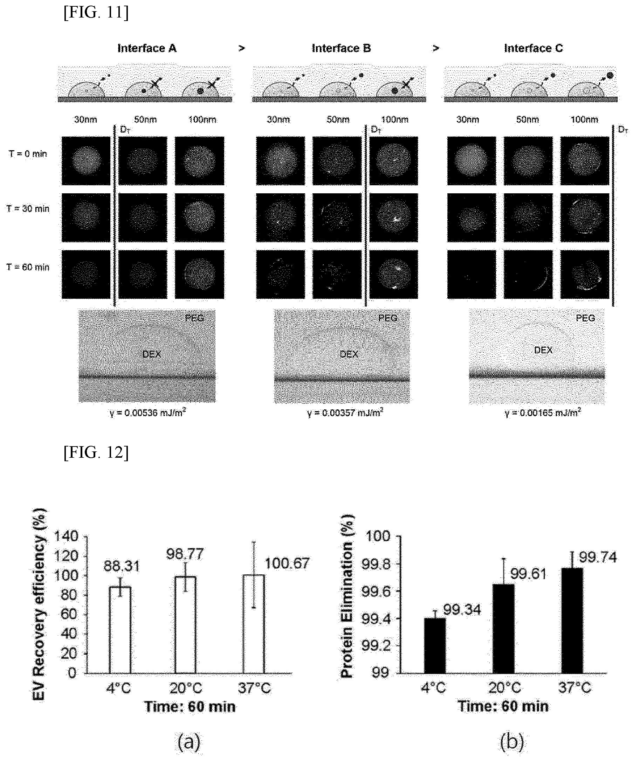

This separation process may be performed in two processes as shown in FIG. 11.

(a) Bio-nanoparticle size > impurity size

When the bio-nanoparticles have a larger particle size than the impurities, the impurities having a relatively small particle size pass through the interface and are transferred to the second aqueous solution phase, and only the bio-nanoparticles remain in the first aqueous solution phase. Next, the bio-nanoparticles may be recovered by separating the first aqueous solution phase from the aqueous two-phase system using equipment such as a pipette or a dropper.

For example, in a biological specimen such as blood, extracellular vesicles (e.g., exosomes), proteins, cholesterol (LDL, HDL), etc. exist. One of the extracellular vesicles, the exosome, has a size of about 100 nm, and the remaining impurities such as proteins or cholesterol have a size of 50 nm or less. Therefore, when designing a composition having interfacial tension to have a critical particle diameter of 50 nm or more at the interface using the aqueous two-phase system phase separation composition presented in the present disclosure, the exosomes remain in the first aqueous solution, and the remaining impurities (protein, cholesterol, etc.) move to the second aqueous solution phase, and only pure exosomes may be selectively isolated.

In particular, the aqueous two-phase system phase separation composition of the present disclosure enables the separation even from particles having a difference of 10 nm in particle size, enabling the separation of extracellular vesicles with high purity.

(b) Bio-nanoparticle size < impurity size

When the bio-nanoparticles have a smaller particle size than the impurities, the bio-nanoparticles having a relatively small particle size pass through the interface and are transferred to the second aqueous solution phase, and only the impurities remain in the first aqueous solution phase. Next, the bio-nanoparticles may be recovered by separating the second aqueous solution phase from the aqueous two-phase system using equipment such as a pipette or a dropper.

This separation process is performed so that the bio-nanoparticles may be sufficiently recovered, and it is carried out within 2 hours, preferably 1 minute to 1 hour, more preferably 5 minutes to 30 minutes, and most preferably 10 to 25 minutes. This time is advantageous of significantly reducing the time compared to the conventional ultracentrifugation process that takes at least 2 hours or more.

In particular, the previously most representative method for isolating exosomes is the method using ultracentrifugation. However, it is difficult to separate exosomes from plasma with high purity and efficiency because the exosomes are small in size at hundreds of nm and do not have a significant difference in density from proteins. In addition, when the ultracentrifuge is used, since the gravitational acceleration actually received by the cells reaches up to 100,000 g, and there is a high possibility of damaging the cells, the method according to the present disclosure may solve problems related to the previous method.

The biological specimen present in the recovered first aqueous solution phase or the second aqueous solution phase may be studied as it is or applied to various fields such as clinical diagnosis or treatment.

In addition, in order to increase the separation capability and the separation rate upon the separation process, temperature or ultrasonic may be applied.

Temperature is a parameter related to the separation rate, and as the temperature is increased, it brings about the effect of increasing the separation rate of bio-nanoparticles and impurities.

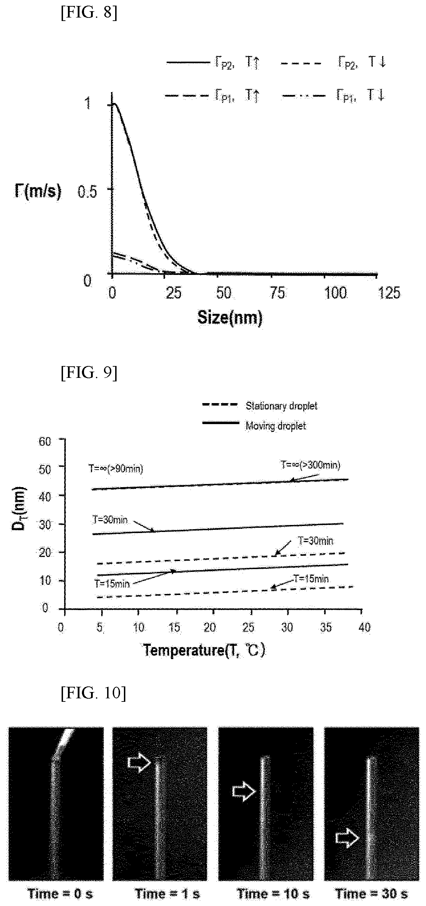

FIG. 8 is a graph showing a change in the escape velocity at the interface according to the particle size. In FIG. 8, $\Gamma_{P1}$: means ratios of particles escaping to the first aqueous solution phase at the interface, $\Gamma_{P2}$: means ratios of particles escaping to the second aqueous solution phase at the interface.

Referring to FIG. 8, as the temperature increases, the Brownian motion of the particles is accelerated, so that the escape velocity of the particles at the interface increases. For example, when the particle size of the impurity in the biological specimen is smaller than the size of the bio-nanoparticle, it means that the rate at which the impurity escapes may be increased. From these results, it can be seen that temperature may be used as a variable of process conditions that may be usefully used when it is necessary to separate in a very accurate size within a short time.

FIG. 9 is a graph showing a change in a critical particle diameter according to a change in temperature.

Referring to FIG. 9, it can be seen that the critical particle diameter tends to linearly increase as the temperature increases. However, the difference in the size of the critical particle diameter is within 10 nm, so even if the temperature is increased upon separation of impurities and bio nanoparticles, it only increases the separation rate and does not increase the critical particle diameter more than necessary, and thus it can be seen that impurities and bio nanoparticles may be separated with high purity.

In addition, it can be seen that, when the first aqueous solution phase P1 is a stationary droplet and a moving droplet, only the separation rate is changed and the critical particle size is finally the same.

In addition, through ultrasonic application, an external physical force lowers the energy barrier of the interface to change the critical particle size or to help the particles to diffuse, thereby increasing the separation rate of impurities and bio-nanoparticles.

Impurities with different particle sizes and bio-nanoparticles have differences in mechanical properties such as differences in size. When the material or composition in addition to the above size is different, mechanical properties such as material, density, and compressibility are greatly different. Here, when ultrasound is applied to the mixed nanoparticles, the nanoparticles have different acoustic radiation forces depending on the particle size, and the nanoparticles move along the sound pressure node line of the ultrasound. That is, when impurities and bio-nanoparticles are mixed, they may be separated and their movement velocity may be increased. In particular, it is possible to increase the separation rate between the impurities and the bio-nanoparticles trapped at the interface between the first aqueous solution phase P1 and the second aqueous solution phase P2, thereby preventing a decrease in the separation rate due to the trapped particles.

This acoustic radiation force may be adjusted by controlling the frequency. Preferably, in the present disclosure, it is preferable carried out for 1 minute to 240 minutes at an intensity of 200 W to 400 W of 0.01 to 100 kHz. In this case, if the intensity of the applied ultrasonic is too strong, it affects the first aqueous solution phase P1 that should be in a bulk form, and the first aqueous solution phase P1 may form fine droplets, so it is properly performed within the above range. Also, the ultrasound application may be performed through an ultrasound generator.

**Application field**

The bio-nanoparticles separated as mentioned above may be applied to various fields.

In an example, bio-nanoparticles as a biomarker may find a use in various fields such as, but not limited to, analytical, diagnostic, and therapeutic uses in biology and medicine.

In particular, the analysis of cells or specimen from a patient may be diagnostically (e.g., for identifying a patient with a specific disease, such as cancer, sepsis, arteriosclerosis and rheumatoid arthritis, etc., a patient exposed to a specific toxin, or patients who respond well to specific treatment or organ transplant) and predictively (e.g., for identifying patients susceptible to developing a particular disease, patients responsive to a particular treatment, or patients receiving a particular organ transplant) adopted. Such methods may facilitate accurate and reliable analysis as multiple (e.g., potentially infinite number) biomarkers from the same biological specimen.

**Mode for Invention**

[Examples]

Hereinafter, the configuration and operation of the present disclosure will be described in more detail through preferred embodiments of the present disclosure. However, this is presented as a preferred example of the present disclosure and may not be construed as limiting the present disclosure in any sense.

**Experimental Example 1: Simulation test**

In addition to theoretical considerations related to the interfacial tension, energy barrier, and critical particle diameter, through simulation results, a direct experiment was performed to determine whether the separation of nanoparticles in the present disclosure may be applied in an actual process.

First, three types of fluorescent beads of 30 nm, 50 nm and 100 nm were prepared as mixed nanoparticles, an aqueous dextran solution was used as the first aqueous solution phase P1, and polyethylene glycol (PEG) was used as the second aqueous solution phase P2. It was performed under conditions of separation coefficient (K, partition coefficient) = 100, temperature = 20°C, time = 300s, boundary tension = 0.013 mJ/m$^2$.

At this time, by controlling the concentrations of the first aqueous solution phase P1 and the second aqueous solution phase P2, an aqueous solution two-phase filter having interfacial tension was designed as follows.

[Table 2]

| Constitution | Interfacial tension (X$10^{-6}$ J/m$^2$) |
| --- | --- |
| A | 5.36 |
| B | 3.57 |
| C | 1.65 |
| D | 30 |
| E | 55 |

A total of 9 tubular test tubes were prepared, and three types of fluorescent beads of 30 nm, 50 nm, and 100 nm were respectively injected into the filters of components A, B, and C, and the movement of the fluorescent beads was confirmed with a fluorescence microscope. At this time, the filter having the configuration of D and E has a critical particle diameter of 30 nm or less without an escape of nanoparticles, and is not shown in FIG. 11. For the measurement, first, 75 $\mu$L of polyethylene glycol aqueous solution forming the second aqueous solution phase P2 was injected into a tubular test tube (inner diameter 2 mm). 5 ng of fluorescent beads were added to 3 $\mu$L of the dextran aqueous solution in the first aqueous solution with uniform mix. 3 $\mu$L of the obtained dispersion was slowly injected into the upper part of the tubular test tube containing the second aqueous solution phase P2. As shown in FIG. 10, it can be seen that the first aqueous solution phase P1 is transferred to the lower side of the test tube.

Immediately after mixing the first aqueous solution phase P1 and the second aqueous solution phase P2 (T=0 min), after 30 minutes and 60 minutes, the movement of the fluorescent beads was measured through a fluorescence spectrometer, and the results are shown in FIG. 11.

FIG.1 is an image showing the degree of movement of fluorescent beads with time, and at the bottom thereof, the interfacial tension between the first aqueous solution phase (DEX) and the second aqueous solution phase (PEG) of A, B, and C is shown.

Referring to the case of composition A of FIG.11, when the interfacial tension is 5.36 $\times10^{-6}$ J/m$^2$, it can be seen that only 30 nm nanoparticles among the nanoparticles of 30 nm, 50 nm and 100 nm moved to the second aqueous solution phase P2. Similarly, in the case of composition B, it can be seen that the interfacial tension is 3.57 $\times10^{-6}$ J/m$^2$, 30 nm and 50 nm nanoparticles move to the second aqueous solution phase P2, and 100 nm nanoparticles remain in the first aqueous solution phase P1. In addition, in the case of composition C, it can be seen that the interfacial tension is 1.65 $\times10^{-6}$ J/m$^2$, and all of 30 nm, 50 nm and 100 nm move to the second aqueous solution phase P2. Through this, as shown in Equation 1, it can be seen that the interfacial tension should be at least 2 $\times10^{-6}$ J/m$^2$ or more.

Through these results, it can be seen that the critical particle diameter that may pass when the nanoparticles are separated may be adjusted by controlling the interfacial tension.

**Experimental Example 2: Separation of protein/extracellular vesicles using aqueous two-phase system phase separation composition**

(1) Preparation of dextran/polyethylene glycol aqueous two-phase system phase separation composition

To make an aqueous two-phase system, polyethylene glycol and dextran were dissolved in phosphate buffered saline (PBS, Phosphate buffered saline) to prepare at concentrations of 10.5% by weight and 45% by weight, respectively. At this time, the interfacial tension between the aqueous solution of dextran/aqueous solution of polyethylene glycol was 5.36$\times10^{-6}$ J/m$^2$. As a result of calculating the separation coefficient and size of the extracellular vesicle and protein, the aqueous two-phase system phase separation composition having the surface tension was calculated to have a critical particle diameter of 42 nm ($\pm$ 5 nm), and due to this, only proteins with a small particle size escape from the interface and are removed, and extracellular vesicles may be recovered with high purity.

Specifically, a biological specimen wherein cell debris is removed from plasma through pre-cleaning was used, and at this time, a specimen containing extracellular vesicles at a concentration of 100 µg/ml and protein at a concentration of 2,000 µg/ml was used.

(2) Separation of extracellular vesicles using aqueous two-phase system phase separation composition

5 ml of polyethylene glycol aqueous solution was added to the horizontal * vertical test tube. Then, 500 µl of the biological specimen was mixed with 0.5 ml of an aqueous dextran solution and dissolved for 1 hour, and then the resulting solution was added to the test tube to induce dextran/polyethylene glycol phase separation.

Then, after extracting the extracellular vesicle, the concentration of the recovered extracellular vesicle and the concentration of the removed protein were measured.

In order to compare the recovery efficiency of the extracellular vesicles, the amount isolated compared to the amount of the total extracellular vesicle or protein in the initial stage was confirmed. To this end, the percentage (%) of the amount of isolated extracellular vesicles or protein with respect to the total amount was defined as recovery efficiency (E), and the recovery efficiency of the aqueous two-phase system was calculated according to Equation 2 below. At this time, the amount of the extracellular vesicle was measured as the amount of RNA, and the amount of protein was measured using a Bradford assay.

[Equation 2]

$$\text{Recovery efficacy (E)} = \frac{\text{Amount of protein or extracellular vesicle isolated on dextran}}{\text{Amount of protein or extracellular vesicle of total solution}} \times 100(\%)$$

(3) Analysis result: Temperature parameter

In order to confirm the separation capability of protein/extracellular vesicles according to temperature change, separation was performed at 4°C, 20°C, and 37°C, and the results obtained were shown in FIG. 12.

FIG. 12 is a graph showing the change of separation of extracellular vesicle and protein according to temperature, with (a) the recovery efficiency of the extracellular vesicle, and (b) the protein removal rate.

Referring to FIG. 12a and 12b, the recovery efficiency of the extracellular vesicles showed a tendency to increase according to the temperature, and to correspond to this, the removal rate of the protein also showed a tendency to increase according to the temperature. Specifically, referring to FIG. 12a, the extracellular vesicles could be recovered without loss, and there was no significant change depending on the temperature in terms of the results at 20°C and 37°C. However, referring to FIG. 12b, the removal rate of protein showed a tendency to increase according to temperature.

Through these results, it can be seen that an increase of the temperature upon separation within the same time may increase the movement speed of the protein, thereby recovering the extracellular vesicles with high purity.

(4) Analysis result: Time parameter

In order to check the separation capability of protein/extracellular vesicle over time, the separation process was performed for 15 minutes, 30 minutes, and 60 minutes at a fixed temperature (20°C).

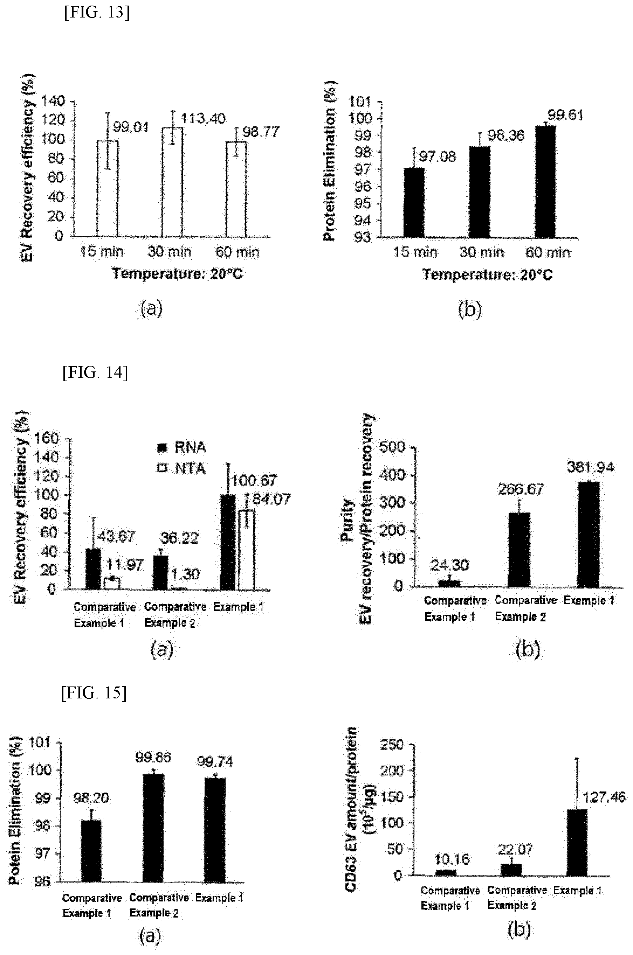

FIG. 13 is a graph showing the change of separation of extracellular vesicle and protein according to time, with (a) the recovery efficiency of the extracellular vesicle, and (b) the removal rate of protein.

Referring to FIGS. 13a and 13b, it can be seen that, although the recovery efficiency of the extracellular vesicles did not change significantly with time, the removal rate of the protein was greatly increased. In particular, referring to FIG. 13b, it can be seen that most of 90% or more of the protein may be removed within 15 minutes.

**Experimental Example 3: Evaluation of extracellular vesicle separation performance according to the separation method**

The recovery efficiency and protein removal rate of the extracellular vesicles according to the protein/extracellular vesicle separation method were measured.

At this time, using the phase separation composition of dextran/polyethylene glycol aqueous two-phase system in Example 1, it was carried out at 20° C for 60 minutes. Also, in Comparative Example 1, ultracentrifugation was performed once for 2 hours at 4°C with 1,000 X g-force, and in Comparative Example 2, ultracentrifugation was performed twice for 4 hours.

FIG. 14a is a graph showing changes in the amount of RNA or the number of particles (NTA) related to the recovery efficiency of extracellular vesicles according to the methods of Example 1, Comparative Example 1 and Comparative Example 2, and FIG. 14b is a graph showing purity.

Referring to FIGS. 14a and 14b, the method using the aqueous two-phase system phase separation composition according to the present disclosure may recover high-purity extracellular vesicles at a higher content than the ultracentrifugation method. In particular, it can be seen that the protein may be separated without damage and loss of the recovered extracellular vesicles because a mechanical force such as ultracentrifugation is not applied.

FIG. 15a is a graph showing the protein removal rates according to the methods of Example 1, Comparative Example 1, and Comparative Example 2, and FIG. 15b is a graph showing the results of measuring the number of extracellular vesicles per ug of protein by CD63 ELISA and Bradford assay. The amount of CD63 indicates the amount of extracellular vesicle, and Bradford assay indicates the amount of total protein. The total protein includes the extracellular vesicle membrane protein and the remaining free proteins, and it means that the higher the amount of CD63 per protein, the higher the purity of the extracellular vesicle was isolated.

Referring to FIGS. 15a and 15b, it can be seen that the method for obtaining the highest purity extracellular vesicles is the Example.

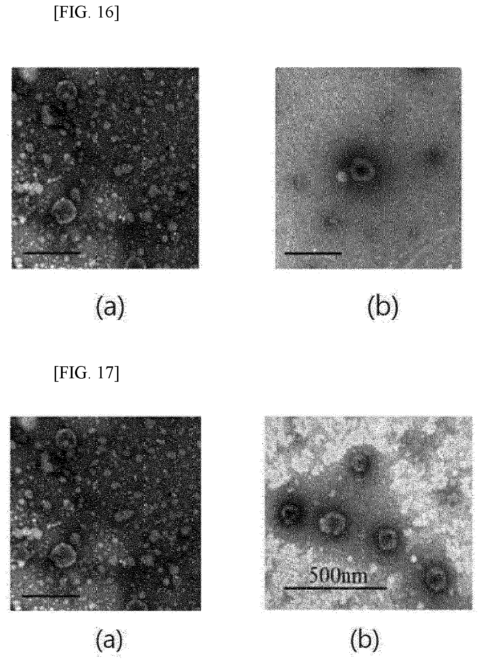

Also, in FIG. 16, (a) is an extracellular vesicle recovered in Example 1, and (b) is an image obtained by photographing the extracellular vesicle recovered in Comparative Example 1 with a transmission electron microscope. In FIG.16, it was possible to confirm the shape of the extracellular vesicles isolated in example.

Combining the results of FIGS. 14 to 16 as described above, it was confirmed that the separation of the extracellular vesicles using the aqueous two-phase system phase separation composition according to the present disclosure is superior in terms of the recovery efficiency and purity of the extracellular vesicles compared to the conventional ultracentrifugation process. In addition, the method using the aqueous two-phase system phase separation composition enables a separation within a shorter time (Example 1: 60 minutes, Comparative Example 1: 2 hours, Comparative Example 2: 4 hours), and it has the advantage of being convenient because it is automatically separated only even though the specimen to be separated drops into the tube.

**Experimental Example 4: Separation performance evaluation using aqueous two-phase system phase separation composition**

Protein/extracellular vesicles were isolated using the method according to the present disclosure and a method of performing agitation/centrifugation, and recovery efficiency and damage were confirmed.

(1) Recovery efficiency

In this case, as Comparative Example 3, it was performed using a method based on Korean Patent Laid-Open Publication No. 10-2016-0116802. Specifically, 500 $\mu$l of a specimen wherein the extracellular vesicles and proteins are mixed was dissolved in an aqueous dextran solution (4.5% by weight) at room temperature for about 1 hour, and then mixed with an aqueous polyethylene glycol solution (10.5% by weight). Then, phase separation was induced by centrifugation for 10 minutes at room temperature with 1,000 $\times$ g-force. At this time, in the specimen, the concentration of the extracellular vesicles was 100 $\mu$g/ml, and the protein concentration was 2,000 $\mu$/ml.

[Table 3]

| | Example 1. | Comparative Example 3 |
|---|---|---|
| Recovery Efficiency of extracellular vesicle | 100.67% | 52.2% |
| Removal rate of the protein | 99.74% | 75.3% |
| Purity (recovery efficiency of extracellular vesicle/recovery efficiency of protein) | 387.19 | 2.11 |

Referring to Table 3, in the method according to the present disclosure, the recovery efficiency of the extracellular vesicles was about 2 times higher than in Comparative Example 3. In addition, it may be confirmed that about 20% or more of the protein is further removed in the protein removal rate. Due to this, it can be seen that the purity of the extracellular vesicles finally recovered through Example 1 is higher.

Although the present disclosure as described above has been described with reference to the examples shown in the drawings, this is merely exemplary and those of ordinary skill in the art will understand that various modifications and variations of the examples are possible therefrom. However, such modifications should be considered to be within the technical protection scope of the present disclosure. Therefore, the true technical protection scope of the present disclosure should be determined by the technical spirit of the appended claims.

**Claims**

1. A method for isolating bio nanoparticles from a biological specimen, the method comprising
mixing a first aqueous solution containing a biological specimen and a second aqueous solution to prepare an aqueous two-phase system phase separation composition that is phase-separated in a first aqueous solution phase comprising the first aqueous solution and a second aqueous solution phase comprising the second aqueous solution, and

   (a) remaining the bio nanoparticles in the first aqueous solution phase by moving impurities within the biological specimen into the second aqueous solution phase; or
   (b) moving the bio nanoparticles excluding impurities within the biological specimen into the second aqueous solution phase to separate the bio nanoparticles and impurities in the biological specimen,

   wherein tension ($\gamma$) at the interface where the first aqueous solution phase and the second aqueous solution phase are phase-separated satisfies Equation 1 below:

$$[\text{Equation 1}]$$

$$2 \times 10^{-7} \text{ J/m}^2 \leq \gamma \leq 50 \times 10^{-5} \text{ J/m}^2$$

2. The method according to claim 1, wherein the first aqueous solution phase is fluidized in the second aqueous solution phase that is a continuous phase in a form of bulk.

3. The method according to claim 1, wherein the aqueous two-phase system phase separation composition moves the impurities or the bio nanoparticles in the biological specimen from the first aqueous solution phase to the second aqueous solution phase by a diffusion.

4. The method according to claim 1, wherein step (a) or (b) is carried out without a stirring and ultracentrifugation process.

5. The method according to claim 1, wherein the biological specimen is one selected from the group consisting of cell culture fluid, blood, plasma, serum, intraperitoneal fluid, semen, amniotic fluid, breast milk, saliva, bronchoalveolar fluid, tumor effluent, tears, runny nose and urine.

6. The method according to claim 1, wherein the bio nanoparticles are extracellular vesicles.

7. The method according to claim 6, wherein the extracellular vesicles are one or more selected from the group consisting of exosomes, ectosomes, exovesicles, microvesicles, microparticles, apoptotic bodies, membrane particles, membrane vesicles, exosome-like vesicles, and ectosome-like vesicles.

8. The method according to claim 1, wherein the bio nanoparticles are one or more selected from the group consisting of the biomolecule and heterobiomolecule.

9. The method according to claim 1, wherein the bio nanoparticles have a diameter of 1 to 500 nm.

10. The method according to claim 1, wherein the first aqueous solution phase-the second solution phase is a combination of polymer-polymer or polymer-high concentration salt.

11. The method according to claim 10, wherein the polymer is one or more selected from the group consisting of one hydrophilic polymer selected from the group consisting of polyarginine, polylysine, polyethylene glycol, polypropylene glycol, polyethyleneimine, chitosan, protamine, polyvinyl acetate, hyaluronic acid, chondroitin sulfate, heparin, alginate, hydroxyoxypropyl methylcellulose, gelatin, starch, poly(vinyl methyl ether ether), polyvinylpyrrolidone, and combinations thereof;

one high molecular polysaccharide selected from the group consisting of cyclodextrin, glucose, dextran, mannose, sucrose, trehalose, maltose, ficoll, inositol, mannitol, sorbitol, sucrose-mannitol, glucose-mannitol, trehalose-polyethylene glycol, sucrose-polyethylene glycol, sucrose-dextran and combinations thereof; and combinations thereof.

**12.** The method according to claim 10, wherein the salts are one selected from the group consisting of $(NH_4)_2SO_4$, $Na_2SO_4$, $MgSO_4$, $K_2HPO_4$, $KH_2PO_4$, NaCl, KCl, NaBr, NaI, LiCl, n-$Bu_4NBr$, n-$Pr_4NBr$, $Et_4NBr$, $Mg(OH)_2$, $Ca(OH)_2$, $Na_2CO_3$, $ZnCO_3$, $Ca_3(PO_4)_2$, $ZnCl_2$, $(C_2H_3)_2Zn$, $ZnCO_3$, $CdCh$, $HgCl_2$, $CoCl_2$, $(CaNO_3)_2$, $BaCl_2$, $MgCl_2$, $PbCl_2$, $AlCl_3$, $FeCl_2$, $FeCl_3$, $NiCl_2$, AgCl, $AuCl_3$, $CuCl_2$, sodium dodecyl sulfate, sodium tetradecyl sulfate, dodecyltrimethylammonium bromide, dodecyltrmethylammonium chloride, tetradecyltrimethylammonium bromide, and combinations thereof.

**13.** The method according to claim 1, wherein a concentration of the first aqueous solution and the second aqueous solution is 0.001 to 20 % by weight.

**14.** The method according to claim 1, wherein in (a) and (b), any one or more processes of temperature control or ultrasonic application are further performed together.

[FIG. 1]

(a)　　　　　(b)　　　　　(c)

P1

P2

P1　　P2　　　P1　　P2

[FIG. 2]

Energy Barrier

P1　　Interface　P2

[FIG. 3]

$T = T_0$ $T = T_1$

(a)

(b)

[FIG. 4]

[FIG. 5]

[FIG. 6]

[FIG.7]

[FIG. 8]

[FIG. 9]

[FIG. 10]

[FIG. 11]

[FIG. 12]

(a)

(b)

[FIG. 13]

(a)

(b)

[FIG. 14]

(a)

(b)

[FIG. 15]

(a)

(b)

[FIG. 16]

(a)

(b)

[FIG. 17]

(a)

(b)

## INTERNATIONAL SEARCH REPORT

| International application No. |
| --- |
| **PCT/KR2020/008353** |

| A. | CLASSIFICATION OF SUBJECT MATTER |
| --- | --- |

**C12Q 1/24**(2006.01)i

According to International Patent Classification (IPC) or to both national classification and IPC

| B. | FIELDS SEARCHED |
| --- | --- |

Minimum documentation searched (classification system followed by classification symbols)

C12Q 1/24; C12M 1/00; C12M 1/12; C12N 13/00; C12N 5/071

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수용액(aqueous solution), 이상계(two-phase), 바이오 나노 입자(bionanoparticle), 분리(separation), 계면장력(interfacial tension)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | KR 10-2016-0114336 A (POSTECH RESEARCH AND BUSINESS DEVELOPMENT FOUNDATION) 05 October 2016. See paragraphs [0002], [0010], [0025]-[0028], [0031] and [0067]; example 2; and claims 1 and 10. | 1-14 |
| Y | ATEFI, Ehsan et al. Ultralow interfacial tensions of aqueous two-phase systems measured using drop shape. Langmuir. 2014, vol. 30, pp. 9691-9699. See abstract; and tables 1 and 3. | 1-14 |
| A | KR 10-2016-0116802 A (POSTECH RESEARCH AND BUSINESS DEVELOPMENT FOUNDATION et al.) 10 October 2016. See entire document. | 1-14 |
| A | KR 10-0863466 B1 (KOREA UNIVERSITY RESEARCH AND BUSINESS FOUNDATION) 16 October 2008. See entire document. | 1-14 |

☑ Further documents are listed in the continuation of Box C.      ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **08 October 2020** | **08 October 2020** |

| Name and mailing address of the ISA/KR | Authorized officer |
| --- | --- |
| **Korean Intellectual Property Office** **Government Complex Daejeon Building 4, 189, Cheongsa- ro, Seo-gu, Daejeon, Republic of Korea** **35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2020/008353** |

**C.**      **DOCUMENTS CONSIDERED TO BE RELEVANT**

| Category* | Citation of document, with indication, where appropriate, of the relevant passages | Relevant to claim No. |
| --- | --- | --- |
| A | KR 10-2018-0081354 A (KOREA UNIVERSITY RESEARCH AND BUSINESS FOUNDATION) 16 July 2018. See entire document. | 1-14 |

Form PCT/ISA/210 (second sheet) (July 2019)

**INTERNATIONAL SEARCH REPORT**
Information on patent family members

International application No.

**PCT/KR2020/008353**

| Patent document cited in search report | | | Publication date (day/month/year) | Patent family member(s) | | | Publication date (day/month/year) |
|---|---|---|---|---|---|---|---|
| KR | 10-2016-0114336 | A | 05 October 2016 | KR | 10-1745455 | B1 | 09 June 2017 |
| | | | | US | 10590372 | B2 | 17 March 2020 |
| | | | | US | 2018-0105778 | A1 | 19 April 2018 |
| | | | | WO | 2016-153187 | A1 | 29 September 2016 |
| KR | 10-2016-0116802 | A | 10 October 2016 | CN | 107532195 | A | 02 January 2018 |
| | | | | EP | 3279332 | A4 | 14 November 2018 |
| | | | | EP | 3279332 | A1 | 07 February 2018 |
| | | | | KR | 10-1761680 | B1 | 23 August 2017 |
| | | | | US | 2018-0164197 | A1 | 14 June 2018 |
| | | | | WO | 2016-159520 | A1 | 06 October 2016 |
| KR | 10-0863466 | B1 | 16 October 2008 | None | | | |
| KR | 10-2018-0081354 | A | 16 July 2018 | KR | 10-1892214 | B1 | 27 August 2018 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190096059

- KR 1020160116802

**Non-patent literature cited in the description**

- **HAMTA, AFSHIN et al.** Application of polyethylene glycol based aqueous two-phase systems for extraction of heavy metals. *Journal of Molecular Liquids,* 2017, vol. 231, 20-24

- **JUAN A. ASENJO et al.** *Aqueous two-phase systems for protein separation: A perspective Journal of Chromatography A,* 09 December 2011, vol. 1218 (49), 8826-8835
- **HYUNWOO SHIN et al.** High-yield isolation of extracellular vesicles using an aqueous two-phase system. *Scientific Reports,* 2015, vol. 5